(19) Europäisches Patentamt
European Patent Office
Office européen des brevets

(11) EP 3 958 987 B1

(12) **EUROPEAN PATENT SPECIFICATION**

(45) Date of publication and mention of the grant of the patent:
**29.11.2023 Bulletin 2023/48**

(21) Application number: **20719210.5**

(22) Date of filing: **22.04.2020**

(51) International Patent Classification (IPC):
*A61Q 19/10* (2006.01) *A61Q 5/02* (2006.01)
*A61K 8/49* (2006.01) *A61K 8/368* (2006.01)
*A61K 8/37* (2006.01) *A61K 8/34* (2006.01)
*A61K 8/31* (2006.01)

(52) Cooperative Patent Classification (CPC):
**A61Q 19/10; A61K 8/31; A61K 8/347; A61K 8/368; A61K 8/37; A61K 8/4926; A61K 8/498; A61Q 5/02;** A61K 2800/524; A61K 2800/592; A61K 2800/77

(86) International application number:
**PCT/EP2020/061159**

(87) International publication number:
**WO 2020/216770 (29.10.2020 Gazette 2020/44)**

(54) **ANTIMICROBIAL COMPOSITIONS**

ANTIMIKROBIELLE ZUSAMMENSETZUNGEN

COMPOSITIONS ANTIMICROBIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2019 EP 19170898**

(43) Date of publication of application:
**02.03.2022 Bulletin 2022/09**

(73) Proprietors:
- **UNILEVER GLOBAL IP LIMITED**
  **Wirral**
  **Merseyside CH62 4ZD (GB)**
  Designated Contracting States:
  **CY DE GB IE IT MT RS TR**
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**

(72) Inventors:
- **CAMPBELL-LEE, Stuart**
  **Wirral Merseyside CH63 3JW (GB)**
- **POINTON, Thomas, Richard**
  **Wirral Merseyside CH63 3JW (GB)**

(74) Representative: **Tansley, Sally Elizabeth**
**Unilever Patent Group**
**Bronland 14**
**6708 WH Wageningen (NL)**

(56) References cited:
**EP-A1- 1 264 547** **EP-A1- 1 964 541**
**DE-A1-102015 225 004** **GB-A- 2 354 771**
**US-A1- 2004 213 861** **US-A1- 2013 156 708**
**US-A1- 2018 177 197**

- **DATABASE GNPD [Online] MINTEL; 23 November 2018 (2018-11-23), anonymous: "Germ Protection Bodywash", XP055618474, retrieved from www.gnpd.com Database accession no. 6126987**
- **ANNA HERMAN: "Antimicrobial Ingredients as Preservative Booster and Components of Self-Preserving Cosmetic Products", CURRENT MICROBIOLOGY, vol. 76, no. 6, 12 April 2018 (2018-04-12) , pages 744-754, XP055619588, New York ISSN: 0343-8651, DOI: 10.1007/s00284-018-1492-2**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 958 987 B1

- John Woodruff: "Preservatives 2006?", SPC, 1 September 2006 (2006-09-01), pages 1-5, XP055079702, Retrieved from the Internet: URL:http://www.creative-developments.co.uk /papers/Preservatives 2006.pdf [retrieved on 2013-09-17] & Nn: "Suprapein (TM) MATERIAL SAFETY DATA SHEET", , 6 September 2019 (2019-09-06), pages 1-4, XP055619575, Retrieved from the Internet: URL:https://www.ulprospector.com/documents /991233.pdf?bs=3887&b=92737&st=20&r=eu&in d =personalcare [retrieved on 2019-09-06]
- Nn: "Nipaguard POB PRESERVATIVE BLEND", , 1 January 2013 (2013-01-01), pages 1-2, XP055618350, Retrieved from the Internet: URL:https://www.clariant.com/en/Solutions/ Products/2013/12/09/18/28/Nipaguard-POB [retrieved on 2019-09-04]
- DATABASE GNPD [Online] MINTEL; 22 March 2019 (2019-03-22), anonymous: "Antibacterial Body Wash", XP055618450, retrieved from www.gnpd.com Database accession no. 6421623
- DATABASE GNPD [Online] MINTEL; 26 March 2019 (2019-03-26), anonymous: "2in1 Anti-Dandruff Shampoo + Conditioner", XP055618455, retrieved from www.gnpd.com Database accession no. 6430183
- DATABASE GNPD [Online] MINTEL; 12 October 2018 (2018-10-12), anonymous: "Shampoo", XP055618493, retrieved from www.gnpd.com Database accession no. 6046603
- DATABASE GNPD [Online] MINTEL; 24 April 2018 (2018-04-24), anonymous: "Hydrating Body Moisturiser", XP055618481, retrieved from www.gnpd.com Database accession no. 5620115

## Description

### Field of the Invention

The present invention relates to the area of preservation for personal care, in particular hair care compositions.

### Background

In the personal care and cosmetic industry there is a constant need for preservation chemicals, especially preservation chemicals that are from natural sources, that are abundant and readily available.

A large number of antimicrobial active compounds are already employed in the personal care industry, but alternatives nevertheless continue to be sought. Not all antimicrobial agents have adequate preservation properties and thus the need for new preservation solutions is particularly required. It is to be noted that the substances used in the personal care field must be:

- toxicologically acceptable
- readily tolerated by the skin
- stable
- inexpensive to prepare (i.e. employing standard processes and/or starting from standard precursors)
- easy to formulate (i.e. preferably liquid) and should not be detrimental to the final product.

DE 10 2015 225004 describes cleansing compositions including at least one special preservative combination, used for the cleaning and/or care of skin and/or hair, and specific preservative combination is used for preserving such surfactant-containing cleansing compositions.

EP1964541 describes a preservative composition comprising: a mixture of two to ten of well characterized fragrance raw materials with a cosmetic function, of which at least two are selected from: allyl caproate, benzyl acetate, benzaldehyde, dihydrolsojasmonate, ethyl phenethylacetal, ethyl cinnamate, ethyl methyl phenyl glycidate, ethyl vanillin, 2-heptylcyclopentanone, geranyl acetate, heliotropine, cis-hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate; and are contained in at least 20 percent by weight of said mixture; at least one preservative; and a sequestrant.

The present application has found a combination of antimicrobial chemicals suitable for achieving preservation of personal care compositions.

A product named Lifebuoy Total 10 Germ Protection Bodywash, sold by Hindustan Lever is said to contain inter alia piroctone olamine, sodium benzoate and eugenol, but according to the order of the ingredients on the product label, the sodium benzoate component is present in the composition at a level less than the piroctone olamine. A further commercial product is Rituals Ancient Samurai Hydrating Body Moisturiser, said to from the label contain limonene, eugenol, sodium benzoate and piroctone olamine; however this is not a cleansing product but clearly a leave-on, moisturizing product.

The listing or discussion of an apparently prior published document in this specification should not necessarily be taken as an acknowledgement that the document is part of the state of the art or is common general knowledge.

### Description of the Invention

The present invention relates to a personal care composition according to claim 1.

The invention further relates to a method of preserving a composition according to claim 13

The invention further relates to the use of a combination according to claim 14.

The weight ratio of piroctone to sodium benzoate to the defined fragrance components iii) is from 1:1:1 to 1:130:130, further preferred more is from 1:1:1 to 1:100:100, more preferably from 1:1:1 to 1:75:75, most preferably from 1:1:1 to 1:50:50.

### Detailed Description of the Invention

Compositions of the invention comprise a piroctone compound. The forms of piroctone compound according to the present invention include piroctone acid, primary, secondary and tertiary olamine salts of piroctone acid (such as the diethanolamine and triethanolamine salts), and mixtures thereof, preferably piroctone acid, primary olamine salt of piroctone acid (i.e. piroctone olamine, also known as Octopirox®) and mixtures thereof, more preferably piroctone acid.

The piroctone compound useful in the present invention typically contains the structure defined by Formula (IV):

(IV)

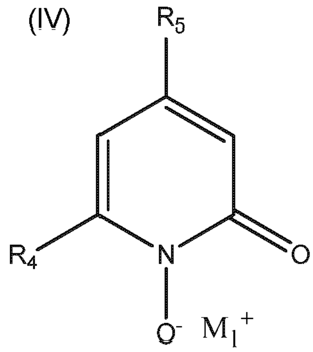

wherein $R_4$ is selected from C1-C17 hydrocarbon radicals, $R_5$ is selected from C1-4 alkyl, C2-4 alkenyl or alkynyl, hydrogen, phenyl or benzyl, and M, is selected from hydrogen, monoethanolamine (MEA), diethanolamine (DEA), or triethanolamine (TEA). Preferred $R_4$ group is $(CH_3)_3CCH_2CH(CH_3)CH_2$- and preferred $R_5$ is a methyl. More preferably, $R_4$ is $(CH_3)_3CCH_2CH(CH_3)CH_2$-, $R_5$ is a methyl and $M_1$ is a hydrogen or MEA. Most preferably, $R_4$ is $(CH_3)_3CCH_2CH(CH_3)CH_2$-, $R_5$ is a methyl and M, is hydrogen.

Typically, the piroctone compound comprises piroctone acid, piroctone olamine or mixtures thereof. The most preferred example is a piroctone acid.

The preferred level of the piroctone compound is from 0.01 to 5 wt% by weight of the total composition, more preferably from 0.05 to 2 wt%, most preferably from 0.1 to 1.5 wt%.

The composition according to the invention comprises sodium benzoate. The preferred level of the sodium benzoate is from 0.01 to 5 wt% by weight of the total composition, more preferably from 0.05 to 2 wt%, most preferably from 0.1 to 1.5 wt%.

Compositions according to the invention comprise a fragrance comprising a fragrance component which is undecalactone. In an embodiment, the undecalactone can be gamma undecalactone. The preferred level of fragrance is from 0.01 to 10 wt% by weight of the total composition, more preferably from 0.05 to 6 wt%, most preferably from 0.05 to 3 wt%.

Preferably the weight ratio of piroctone to sodium benzoate to the defined fragrance components iii) is from 1:1:1 to 1:130:130, further preferred more is from 1:1:1 to 1:100:100 more preferably from 1:1:1 to 1:75:75, most preferably from 1:1:1 to 1:50:50.

Preferably the composition comprises an aqueous base of at least 50 wt% water of the total composition.

In a preferred embodiment, the personal care composition comprises a cleansing phase comprising one or more cleansing surfactants.

The composition according to the invention preferably comprises a surfactant chosen from anionic, nonionic, zwitterionic or amphoteric surfactants or mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alphaolefin sulphonates, especially their sodium, magnesium ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauroyl isethionate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic (C8-C18) primary or secondary linear or branched chain alcohols or phenols with alkylene oxides, usually ethylene oxide and generally having from 6 to 30 ethylene oxide groups. Other suitable nonionic surfactants include mono-or di-alkyl alkanolamides, glycolipids preferably selected from the group of rhamnolipids and sophorolipids.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine, coco mono-or di-ethanolamide, coco mono-isopropanolamide and sodium cocamphopropionate.

Generally, the surfactants are present in shampoo compositions of the invention in an amount of from 0.1 to 50%, preferably from 5 to 30%, more preferably from 10% to 25% by weight.

Compositions of the invention are preferably shampoo compositions.

The compositions of the invention may comprise silicone conditioning agent, preferably in the form of emulsified droplets for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst ($mm^2/s$) at 25°C; the viscosity of the silicone itself is preferably at least 60,000 cst, ($mm^2/s$), most preferably at least 500,000 cst, ($mm^2/s$), ideally at least 1,000,000 cst. ($mm^2/s$). Preferably the viscosity does not exceed $10^9$ est ($mm^2/s$) for ease of formulation.

Emulsified silicones for use in the shampoo compositions of the invention will typically have a D90 silicone droplet size in the composition of less than 30, preferably less than 20, more preferably less than 10 microns, ideally from 0.01 to 1 micron. Silicone emulsions having an average silicone droplet size (D50) of 0.15 microns or less are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions/microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex. Dow Corning).

Suitable quaternary silicone polymers are described in EP-A-0 530,974. A preferred quaternary silicone polymer is K3474, ex. Goldschmidt.

Also suitable are emulsions of amino functional silicone oils with nonionic and/or cationic surfactant. Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex. Dow Corning).

The total amount of silicone is preferably from 0.01 to 10 wt% of the total composition, more preferably from 0.1 to 5 wt%, most preferably 0.5 to 3 wt% is a suitable level. Preferably the weight ratio of silicone conditioning agent to short chain, unsaturated organic, acid is from 20:1 to 1:4.

Compositions of the invention may comprise further ingredients such as cationic deposition polymer preferably those selected from cationic cellulose and cationic guar derivatives.

Other examples of ingredients found in compositions according to the invention comprise fragrances and pH adjusting agents. Preferably the pH of the composition at 20°C is from 3 to 7, more preferably from 4 to 6.

The invention will now be illustrated by the following non-limiting examples.

## Examples

The differing behaviors of inhibitory antimicrobials in isolation and mixtures have been widely explored using the concept of the Fractional Concentration and Fractional Inhibitory Concentration (FIC). See for instance JRW Lambert and R Lambert, J. Appl. Microbiol 95, 734 (2003); T. Jadavji, CG Prober and R Cheung, Antimicrobial Agents and Chemotherapy 26, 91 (1984), and WO 2004/006876. These parameters can be defined as follows:

FIC (component a) = MIC (component a tested in the mixture) / MIC (component a tested as a single active).

The interactions between antimicrobials can be additive, synergistic or possibly antagonistic depending on whether the efficacy of the combination is equivalent to, greater than or less than that obtained for the same total concentration of the individual components when tested alone.

These relationships can be expressed mathematically by summing the FIC values for all the components present in the mixture to give the "fractional inhibitory index":

$$\sum FIC = FIC_{\text{(component 1)}} + FIC_{\text{(component 2)}} + FIC_{\text{(component 3)}}$$

Such that:

ΣFIC ≥ 1 corresponds to additive or antagonistic activity
ΣFIC < 1 corresponds to synergistic activity

A comparable method is the calculation of the synergy index (SI) which is an industrial accepted method described by Kull, F.C.; Eisman, P.C.; Sylwestrowicz,H.D. and Mayer, R.L., in Applied Microbiology 9:538-541 (1961).

Liquid broth assays (MIC and checkerboard) were conducted to identify the minimum concentration(s) of individual and tertiary antimicrobial chemicals. A modified methodology to ISO 20776-1:2006 was utilized for the screening as follows. Stock solutions of antimicrobial chemicals and tryptic soy broth were inoculated with $1\text{-}5\times10^6$ microorganism mixture of Gram negative fermenting bacteria; *Enterobacter gergoviae* and *Klebsiella* species.

Samples were incubated at 30°C for 24 hours, after which optical densities at $OD_{600}$nm were measured. MIC was defined as the concentration at which <25% growth was observed in comparison to a positive growth control containing no antimicrobial chemicals.

Table 1 MIC concentrations were established for the individual chemicals

| **Compound Name** | **MIC (wt%)** |
|---|---|
| Octopirox | 0.063 |
| Sodium benzoate | 0.5 |
| Benzyl Acetate | 2 |
| Geranyl Acetate | 2 |
| Geraniol Natural | 2 |
| Citroneol | 2 |
| Terponeol | 0.125 |
| Menthol | 2 |
| Eugenol | 0.063 |
| Phenethyl Alcohol | 0.5 |
| Gamma Undecalactone | 2 |
| Itaconic Acid | 0.5 |
| Phenoxyethanol | 0.25 |
| Ethyl Butyrate | 2 |
| Hexyl acetate | 2 |
| Limonene | 2 |
| Eucalyptol | 2 |

Tertiary combinations were evaluated at sub-MIC concentrations to identify if synergy was observed.

Table 2: Fractional Inhibitory Concentrations for piroctone olamine, sodium benzoate and a third chemical combinations against Gram negative fermenting bacteria. FIC values are the lowest values observed per tertiary combination.

| **Chemical 1** | **Chemical 2** | **Chemical 3** | **FIC** |
|---|---|---|---|
| Piroctone olamine | Sodium benzoate | Benzvll acetate | 0.13 |
| Piroctone olamine | Sodium benzoate | Hexyl acetate | 0.37 |
| Piroctone olamine | Sodium benzoate | Phenethyl alcohol | 0.50 |
| Piroctone olamine | Sodium benzoate | Ethyl butyrate | 0.56 |
| Piroctone olamine | Sodium benzoate | Eucalyptol | 0.56 |
| Piroctone olamine | Sodium benzoate | Limonene | 0.62 |

(continued)

| Chemical 1 | Chemical 2 | Chemical 3 | FIC |
|---|---|---|---|
| Piroctone olamine | Sodium benzoate | Gamma undecalactone | 0.66 |
| Piroctone olamine | Sodium benzoate | Eugenol | 0.75 |
| Piroctone olamine | Sodium benzoate | Terponeol | 1.00 |
| Piroctone olamine | Sodium benzoate | Phenoxyethanol | 1.12 |
| Piroctone olamine | Sodium benzoate | Geraniol | 1.16 |
| Piroctone olamine | Sodium benzoate | Geranyl acetate | 1.16 |
| Piroctone olamine | Sodium benzoate | Menthol | 1.19 |
| Piroctone olamine | Sodium benzoate | Citroneol | 2.00 |

(only compositions with undecalactone are in accordance with the invention).

In the table above any FIC value of 1.00 or above is non-synergistic or merely additive. A value of 2.00 is antagonistic.

Table 2 demonstrates that the Examples according to the invention demonstrated a synergistic chemical combination against Gram negative fermenting bacteria.

Table 3 demonstrates further the benefits of compositions and methods according to the invention, tested under the same conditions as the compositions in Table 2, with different ingredient component ratios. The following are the tertiary ratio combinations and corresponding FIC scores observed when varied ratios are included.

| Chemical names | | | Ratio of components (wt) | | | |
|---|---|---|---|---|---|---|
| Ingredient A | Ingredient B | Ingredient C | Ingredient A | Ingredient B | Ingredient C | FIC |
| | | | 2 | 32 | 64 | 1.00 |
| | | | 2 | 16 | 128 | 1.00 |
| Octopirox | Sodium benzoate | Geranyl Acetate | 8 | 8 | 16 | 1.19 |
| | | | 4 | 16 | 64 | 1.00 |
| | | | 1 | 64 | 8 | 1.16 |
| Octopirox | Sodium benzoate | Geraniol | 1 | 8 | 1 | 1.75 |
| | | | 8 | 64 | 16 | 2.06 |
| | | | 8 | 8 | 256 | 2.13 |
| Octopirox | Sodium benzoate | Citroneol | 16 | 8 | 8 | 2.16 |
| | | | 4 | 16 | 4 | 1.00 |
| | | | 2 | 16 | 8 | 1.00 |
| Octopirox | Sodium benzoate | Terponeol | 1 | 32 | 8 | 1.13 |
| | | | 4 | 32 | 32 | 1.13 |
| | | | 1 | 64 | 16 | 1.19 |
| Octopirox | Sodium benzoate | Menthol | 2 | 32 | 128 | 1.25 |
| | | | 1 | 16 | 2 | 0.62 |
| | | | 2 | 8 | 2 | 0.62 |
| Octopirox | Sodium benzoate | Eugenol | 2 | 32 | 1 | 0.87 |
| | | | 2 | 8 | 8 | 0.50 |
| | | | 2 | 8 | 4 | 0.44 |
| Octopirox | Sodium benzoate | Phenethyl Alcohol | 1 | 8 | 16 | 0.50 |

(continued)

| Chemical names | | | Ratio of components (wt) | | | |
|---|---|---|---|---|---|---|
| **Ingredient A** | **Ingredient B** | **Ingredient C** | **Ingredient A** | **Ingredient B** | **Ingredient C** | **FIC** |
| | | | 2 | 8 | 32 | 0.50 |
| | | | 2 | 16 | 32 | 0.63 |
| Octopirox | Sodium benzoate | Gamma Undecalactone | 1 | 32 | 16 | 0.69 |
| | | | 4 | 16 | 8 | 1.00 |
| | | | 4 | 16 | 16 | 1.25 |
| Octopirox | Sodium benzoate | Phenoxyethanol | 4 | 8 | 16 | 1.13 |
| | | | 1 | 16 | 16 | 0.44 |
| | | | 1 | 8 | 64 | 0.50 |
| Octopirox | Sodium benzoate | Ethyl butyrate | 2 | 16 | 16 | 0.56 |
| | | | 1 | 8 | 8 | 0.28 |
| | | | 1 | 8 | 32 | 0.38 |
| Octopirox | Sodium benzoate | Hexyl acetate | 1 | 16 | 8 | 0.41 |
| | | | 2 | 16 | 8 | 0.53 |
| | | | 1 | 16 | 64 | 0.63 |
| Octopirox | Sodium benzoate | Limonene | 2 | 8 | 64 | 0.63 |
| | | | 1 | 16 | 16 | 0.44 |
| | | | 1 | 16 | 32 | 0.50 |
| Octopirox | Sodium benzoate | Eucalyptol | 4 | 8 | 8 | 0.66 |
| | | | 1 | 8 | 8 | 0.28 |
| | | | 2 | 8 | 8 | 0.41 |
| Octopirox | Sodium benzoate | Eucalyptol | 1 | 8 | 16 | 0.31 |

(only compositions with undecalactone are in accordance with the invention).

A shampoo was prepared having the formulation according to Table 4.

**Table 4:** Shampoo formulation

| INCI name | Tradename | Composition 1 (wt%) |
|---|---|---|
| Sodium laureth sulphate (2EO) | Texapon N70 | 13 |
| Cocamidoporopyl betaine | Tego Betain CK KB5 | 1 |
| Polyquaternium-10 | UCARE JR 30M | 0.5 |
| Dimethiconol (and) TEA-Dodecylbenzenesulfonate | Xiameter MEM-1.788 | 4 |
| Piroctone acid preservation system according to Table 2 | | 0.75 |
| Water | Water and minors | To 100% |

(only compositions with undecalactone are in accordance with the invention).

## Claims

1. A personal care composition comprising:

i) a piroctone compound;
ii) sodium benzoate;
iii) a fragrance comprising a fragrance component which is undecalactone.

**2.** A personal care composition according to claim 1, in which the piroctone compound is a piroctone acid.

**3.** A personal care composition according to claim or claim 2, in which the ratio of piroctone to sodium benzoate to undecalactone is from 1:1:1 to 1:130:130.

**4.** A personal care composition according to any preceding claim, in which the undecalactone is gamma undecalactone.

**5.** A personal care composition according to any preceding claim, in which the personal care composition comprises a cleansing phase comprising one or more cleansing surfactants.

**6.** A personal care composition according to claim 5, in which the cleansing surfactant is alkyl sulphate and/or ethoxylated alkyl sulphate anionic surfactant; and the surfactant is present at a level of from 0.5 to 20% by weight of the total composition, preferably from 5 to 15 wt%.

**7.** A personal composition according to any of the preceding claims, in which the piroctone compound is present at a level of from 0.01 to 5% by weight of the total composition, preferably from 0.05 to 2 wt%, more preferably from 0.1 to 1 wt%.

**8.** A personal care composition according to any preceding claim, in which the pH at 20°C of the composition is from 3 to 6.

**9.** A personal care composition according to any preceding claim, in which the level of sodium benzoate is from 0.01 to 5% by weight of the total composition.

**10.** A personal care composition according to any preceding claim which is a hair treatment composition.

**11.** A personal care composition according to any preceding claim that is a rinse-off composition.

**12.** A personal care composition according to claim 10 or claim 11 which is a shampoo composition.

**13.** A method of preserving a composition by the use of a combination of:

i) a piroctone compound;
ii) sodium benzoate; and
iii) a fragrance comprising a fragrance component which is undecalactone.

**14.** Use of a combination of:

i) a piroctone compound;
ii) sodium benzoate; and
iii) a fragrance comprising a fragrance component which is undecalactone to preserve a personal care composition.

## Patentansprüche

**1.** Körperpflegezusammensetzung, umfassend:

i) eine Piroctonverbindung;
ii) Natriumbenzoat;
iii) einen Duftstoff, umfassend eine Duftstoffkomponente, welche ein Undecalacton ist.

**2.** Körperpflegezusammensetzung nach Anspruch 1, in der die Piroctonverbindung eine Piroctonsäure ist.

3. Körperpflegezusammensetzung nach Anspruch 1 oder Anspruch 2, in der das Verhältnis von Pirocton zu Natriumbenzoat zu Undecalacton 1:1:1 bis 1:130:130 beträgt.

4. Körperpflegezusammensetzung nach einem vorhergehenden Anspruch, in der das Undecalacton Gamma-Undecalacton ist.

5. Körperpflegezusammensetzung nach einem vorhergehenden Anspruch, in der die Körperpflegezusammensetzung eine Reinigungsphase umfasst, die ein oder mehrere Reinigungstenside umfasst.

6. Körperpflegezusammensetzung nach Anspruch 5, in der das Reinigungstensid Alkylsulfat und/oder anionisches ethoxyliertes Alkylsulfat-Tensid ist und das Tensid in einer Menge von 0,5 bis 20%, bezogen auf das Gewicht der Gesamtzusammensetzung, vorzugsweise von 5 bis 15 Gew.-%, vorliegt.

7. Körperpflegezusammensetzung nach einem der vorhergehenden Ansprüche, in der die Piroctonverbindung in einer Menge von 0,01 bis 5%, bezogen auf das Gewicht der Gesamtzusammensetzung, vorzugsweise von 0,05 bis 2 Gew.-%, besonders bevorzugt von 0,1 bis 1 Gew.-%, vorliegt.

8. Körperpflegezusammensetzung nach einem vorhergehenden Anspruch, in der der pH-Wert der Zusammensetzung bei 20°C 3 bis 6 beträgt.

9. Körperpflegezusammensetzung nach einem vorhergehenden Anspruch, in der die Menge des Natriumbenzoats 0,01 bis 5%, bezogen auf das Gewicht der Gesamtzusammensetzung, beträgt.

10. Körperpflegezusammensetzung nach einem vorhergehenden Anspruch, die eine Haarbehandlungszusammensetzung ist.

11. Körperpflegezusammensetzung nach einem vorhergehenden Anspruch, die eine abspülbare Zusammensetzung ist.

12. Körperpflegezusammensetzung nach Anspruch 10 oder Anspruch 11, die eine Shampoozusammensetzung ist.

13. Verfahren zum Konservieren einer Zusammensetzung durch die Verwendung einer Kombination aus:

    i) einer Piroctonverbindung;
    ii) Natriumbenzoat; und
    iii) einem Duftstoff, umfassend eine Duftstoffkomponente, die Undecalacton ist.

14. Verwendung einer Kombination aus:

    i) einer Piroctonverbindung;
    ii) Natriumbenzoat; und
    iii) einem Duftstoff, umfassend eine Duftstoffkomponente, die Undecalacton ist, zum Konservieren einer Körperpflegezusammensetzung.

## Revendications

1. Composition pour l'hygiène personnelle comprenant :

    i) un composé de piroctone ;
    ii) du benzoate de sodium ;
    iii) un parfum comprenant un constituant de parfum lequel est l'undécalactone.

2. Composition pour l'hygiène personnelle selon la revendication 1, dans laquelle le composé de piroctone est un acide de piroctone.

3. Composition pour l'hygiène personnelle selon la revendication 1 ou revendication 2, dans laquelle le rapport de piroctone à benzoate de sodium à undécalactone est de 1:1:1 à 1:130:130.

**4.** Composition pour l'hygiène personnelle selon l'une quelconque des revendications précédentes, dans laquelle l'undécalactone est la gamma undécalactone.

**5.** Composition pour l'hygiène personnelle selon l'une quelconque des revendications précédentes, dans laquelle la composition pour l'hygiène personnelle comprend une phase de nettoyage comprenant un ou plusieurs tensioactifs de nettoyage.

**6.** Composition pour l'hygiène personnelle selon la revendication 5, dans laquelle le tensioactif de nettoyage est un tensioactif anionique de sulfate d'alkyle et/ou sulfate d'alkyle éthoxylé ; et le tensioactif est présent à une teneur de 0,5 à 20 % en masse de la composition totale, de préférence de 5 à 15 % en masse.

**7.** Composition pour l'hygiène personnelle selon l'une quelconque des revendications précédentes, dans laquelle le composé de piroctone est présent à une teneur de 0,01 à 5 % en masse de la composition totale, de préférence de 0,05 à 2 % en masse, encore mieux de 0,1 à 1 % en masse.

**8.** Composition pour l'hygiène personnelle selon l'une quelconque des revendications précédentes, dans laquelle le pH à 20°C de la composition est de 3 à 6.

**9.** Composition pour l'hygiène personnelle selon l'une quelconque des revendications précédentes, dans laquelle la teneur en benzoate de sodium est de 0,01 à 5 % en masse de la composition totale.

**10.** Composition pour l'hygiène personnelle selon l'une quelconque des revendications précédentes qui est une composition pour le traitement des cheveux.

**11.** Composition pour l'hygiène personnelle selon l'une quelconque des revendications précédentes qui est une composition à rincer.

**12.** Composition pour l'hygiène personnelle selon la revendication 10 ou revendication 11 qui est une composition de shampoing.

**13.** Procédé de conservation d'une composition par l'utilisation d'une combinaison de :

i) un composé de piroctone ;
ii) du benzoate de sodium ; et
iii) un parfum comprenant un constituant de parfum qui est l'undécalactone.

**14.** Utilisation d'une combinaison de :

i) un composé de piroctone ;
ii) du benzoate de sodium ; et
iii) un parfum comprenant un constituant de parfum qui est l'undécalactone pour conserver une composition pour l'hygiène personnelle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102015225004
- EP 1964541 A
- WO 9631188 A
- EP 0530974 A
- WO 2004006876 A

### Non-patent literature cited in the description


- **JRW LAMBERT ; R LAMBERT.** *J. Appl. Microbiol,* 2003, vol. 95, 734
- **T. JADAVJI ; CG PROBER ; R CHEUNG.** *Antimicrobial Agents and Chemotherapy,* 1984, vol. 26, 91
- **KULL, F.C. ; EISMAN, P.C. ; SYLWESTROWICZ,H.D. ; MAYER, R.L.** *Applied Microbiology,* 1961, vol. 9, 538-541